(19) Europäisches Patentamt

European Patent Office

Office européen des brevets

(11) Publication number: **0 257 923 B1**

(12) ## EUROPEAN PATENT SPECIFICATION

(45) Date of publication of patent specification: **22.01.92** (51) Int. Cl.⁵: **A61L 27/00**

(21) Application number: **87307136.9**

(22) Date of filing: **12.08.87**

(54) **Improvements in prostheses.**

(30) Priority: **22.08.86 GB 8620469**

(43) Date of publication of application:
**02.03.88 Bulletin 88/09**

(45) Publication of the grant of the patent:
**22.01.92 Bulletin 92/04**

(84) Designated Contracting States:
**CH DE FR IT LI NL SE**

(56) References cited:
**WO-A-81/02667**
**WO-A-81/02668**

(73) Proprietor: **UNITED KINGDOM ATOMIC ENER-GY AUTHORITY**
**11 Charles II Street**
**London SW1Y 4OP(GB)**

(72) Inventor: **Dearnaley, Geoffrey**
**11 Clifton Drive**
**Abingdon Oxon(GB)**

(74) Representative: **Wood, Paul Austin**
**Patents Branch United Kingdom Atomic En-ergy Authority 11 Charles II Street**
**London SW1Y 4OP(GB)**

Rank Xerox (UK) Business Services

## Description

The present invention relates to prostheses, that is to say artificial replacements for components of the human body which may be deficient in some way, or missing, even.

The introduction of prostheses such as hip or knee replacements, support pins or plates etc. into human patients is being carried out at an increasing rate. It is a recognised fact that, once implanted, these components undergo wear, generally by a corrosion/erosion mechanism by which, for example, the sliding of muscle over the surface will result in an erosion of the prostheses. In many cases, such as hip or knee replacements, wear of the bearing surfaces of the component is highly undesirable since it leads to a mechanical failure and is accompanied by the release of foreign material into the body tissue.

For this reason, care must be exercised in the choice of materials for biomedical use, since elements such as nickel and chromium are now recognised as being potentially harmful if released into body tissues by either corrosion or corrosion/erosion. Certain alloys, such as those of titanium, are therefore increasingly favoured since they are not only highly corrosion resistant but also appear to possess a high degree of biocompatibility, which is an important factor if bone ingrowth is to be used, for example, to lock the component in place without resort to cement. Ceramics, such as alumina, are being developed as wear resistant and innocuous materials for these applications.

However, the site of a prosthetic implant is frequently one at which inflammation and post-operative infection is observed to occur. This may result partly from the effects of wear debris, cement particles, or other foreign matter which the body attempts to reject. The local trauma associated with this may cause a variation in pH (acidity) that further exacerbates corrosion, especially if the design of the prosthesis is one in which crevices occur, or small amplitude mechanical movement takes place, i.e. such as to cause fretting. Much effort has been devoted to the development of corrosion-resistant and wear-resistant materials for these applications, e.g. titanium alloys, alumina.

The situation has been reached, as a result of these endeavours, by which the wear due to corrosion/erosion mechanisms, is relatively low, but nevertheless there is still concern about the consequences of ion release and debris production within body tissues, which may lead to localised inflammation and infection sites.

Certain elements such as silver and gold, are well known to have a broad spectrum biocidal action within the human body. For example, the use of silver compounds for the cauterisation of wounds has been known since classical times. However, these elements are costly, and are not easily introduced into those materials which are commonly used for prostheses. Conventional plating techniques, with their risk of spalling under the arduous chemical and physical conditions, are not satisfactory.

According to the present invention there is provided a prosthesis having a surface region which in use will be subject to corrosion or erosion which has implanted within it a material adapted to have a beneficial biocidal action when released into the body of a recipient of the prostheses as a result of the corrosion or erosion of the said surface region of the prosthesis.

Suitable biocidally active materials are noble metals, in particular gold and silver, which may be implanted by bombarding appropriate parts of prostheses with a beam of the appropriate ions having energies of some 100 keV until an ion dose of at least $10^{15}$ to $10^{16}$ ions/cm$^2$ has been implanted. Alternatively, the related processes of ion beam mixing or radiation enhanced diffusion can be employed. In these processes an initially deposited layer is caused to migrate into, and possibly react with, a substrate by means of a subsequent ion bombardment. Reaction between the initial layer and the bombarding species also may be arranged.

The invention will now be further described and explained, by way of example, with reference to the accompanying drawing which shows diagrammatically the operations involved in producing a component for a replacement hip joint embodying the invention.

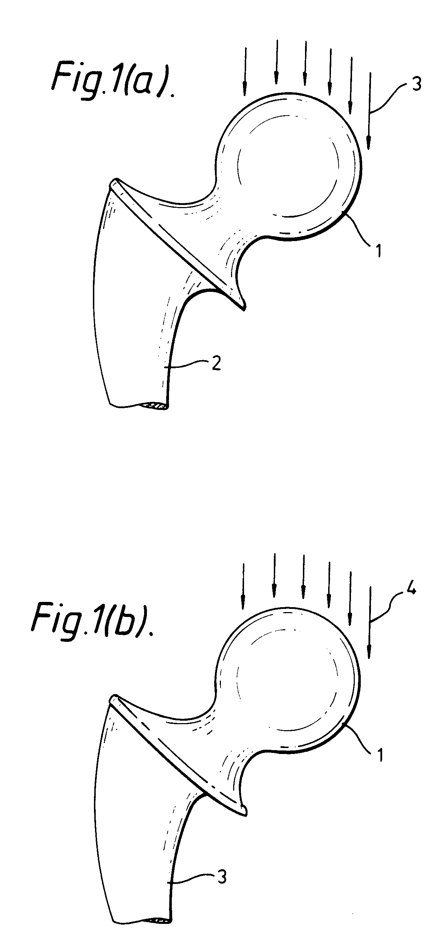

Referring to the drawing, the femoral component of a replacement hip joint prosthesis consists of a spherical head 1 which is attached to, but offset from the longitudinal axis of, a tapering shank 2. Conventionally, such femoral components are made from an alloy of titanium. In carrying out the present invention, the femoral component is subjected first to a bombardment by a beam 3 of ions of silver having an energy of the order of tens of kiloelectron volts until an ion dose of some $10^{15}$ $10^{16}$ ions/cm$^2$ is implanted, as shown in Figure 1-(a). It is then subjected to a second bombardment with a beam 4 of ions of nitrogen at energies of 10 kiloelectron volts or above at temperatures of about 500 °C until a nitrogen ion dose of about $1-5 \times 10^{17}$ ions/cm$^2$ has been implanted, as shown in Figure 1(b). Alternatively, the ion bombardments can be carried out simultaneously.

The process of ion implantation provides a highly controllable method for the introduction of a determined quantity of material, irrespective of the normal solubility or diffusivity, into the surface regions of a material, as do the physically related methods of implantation involving ion beam mixing and radiation enhanced diffusion.

It is clearly important to be able to relate the depth to which the beneficial species, e.g. silver, is introduced to the rate at which the material surface is eroded or corroded within the body. It is furthermore desirable that the volume of material released into body tissues is held to a minimum. By ion implantation, and the related processes of ion beam mixing of thin coatings, or radiation enhanced diffusion, the depth to which the beneficial species is introduced can be controlled to a high degree.

Furthermore, the introduction and subsequent release of bio-active material into the body is controlled both by the implanted ions and the temperature of implantation. Elements such as silver are known to be particularly effective as biocidal agents if they are present in a soluble or finely-divided form. It is an advantage of ion implantation that the additive atoms are very finely dispersed within the host material.

Likewise, gold is known to have a beneficial effect not only in regard to the inflammatory process, but also in conditions such as rheumatoid arthritis, possibly by virtue of an interaction with the processes of prostaglandin generation.

Since the depth of penetration which is achievable by the technique of ion implantation or ion beam mixing is limited (generally being less than, or of the order of, one micron) it is also necessary to arrange that the wear rate of the prosthetic component is both small and predictable. For this reason it may be necessary to implant the surface of prostheses made of titanium alloys with nitrogen or carbon, so as to create a fine dispersion of second-phase TiN or TiC which will strengthen and harden the material in order that it may resist wear by oxidised or work-hardened debris which may have become embedded in the surface of a mating component, e.g. an acetabular cup of ultra-high molecular weight polyethylene.

The depth of penetration of the ion implanted species, e.g. silver, also can be increased by the mechanism of radiation enhanced diffusion, and it is particularly relevant that such defect-enhanced diffusion takes place predominantly for those species, such as silver or gold, which possess relatively high thermal diffusion coefficients in many matrix materials. Such radiation-enhanced diffusion is influenced by the temperature of the ion bombardment and the dose rate or rate of arrival of ions. Enhanced diffusion of silver has been observed in other substances, such as beryllium, and is expected to occur in many other substances. The point defects (vacancies or interstitials) which enhance the diffusion (sometimes by three of more orders of magnitude) are created by the energetic ion bombardment, i.e. at energies of 10 keV or above, (i.e. well above the displacement energy of atoms in matter, which is of the order of 25 eV). Preferably the temperature should be above that at which vacancies are mobile e.g. in titanium this is above 480° C. Suitable ions for this process are light ions such as $A^+$, $B^+$, $N^+$ or $C^+$. When $N^+$ or $C^+$ ions are used in conjunction with titanium prosthesis, then the enhanced diffusion of the biocidally active material and the formation of second-phase TiN or TiC can be achieved simultaneously.

It will not normally be necessary to treat the entire area of the prosthesis by these processes. Clinical experience can suggest which regions are most likely to become prone to inflammation or infection. For example, there may be sites associated with the production of debris by wear or fretting of the prosthesis itself, or they may be associated with the rubbing or sliding of a muscle over the prosthesis. It is desirable to localise the treatment of ion implantation to such specific areas, and this is achievable very readily by masking the irradiation from the line-of-sight influence of the energetic ion beam.

## Claims

1.  A method of manufacturing a prosthesis having a surface region which in use will be subject to corrosion or erosion wherein there is included the operations of subjecting the said region of the prosthesis to bombardment by ions of a material such as to have a beneficial biocidal action when released from the surface region of the prosthesis by corrosion or erosion thereof in use until a dose of at least $10^{15}$ ions/cm$^2$ of the said material has been implanted into the said region of the prosthesis and then subjecting the implanted region of the prosthesis to bombardment by ions of a second species such as to cause the ions of the said material to migrate into the prosthesis.

2.  A method according to claim 2 wherein the second ion species has an energy greater than 10 KeV and the second bombardment is continued until an ion dose at least equal to that of the said material as been implanted.

3.  A method according to claim 1 or claim 2 wherein the second bombardment is carried out at a temperature at which vacancies in the crystal lattice of the material of which the prosthesis is made are mobile.

4.  A method according to any of claims 1 to 2 wherein the bombardments are carried out simultaneously.

5. A method according to any preceding claim wherein the said material is a noble metal.

6. A method according to any preceding claim wherein the material is silver or gold.

7. A method according to any preceding claim wherein the second ion species is a light reactive ion species.

8. A method according to claim 7 wherein the second ion species is such as to be capable of reacting with the material of the prosthesis.

9. A method according to claim 8 wherein the prosthesis is made of titanium.

10. A method according to any preceding claim wherein the second ion species is N$^+$ or C$^+$.

**Revendications**

1. Procédé de fabrication d'une prothèse ayant une région superficielle qui, pendant l'utilisation, est soumise à une corrosion ou une érosion, dans lequel sont comprises des opérations de traitement de la région de la prothèse par un bombardement par des ions d'une matière qui, lorsqu'elle est libérée par la région de la surface de la prothèse par corrosion ou érosion de cette surface pendant l'utilisation, a une action biocide avantageuse, jusqu'à l'implantation d'une dose d'au moins 10$^{15}$ ions/cm$^2$ de la matière dans ladite région de la prothèse, puis de traitement de la région implantée de la prothèse par bombardement par des ions d'une seconde espèce afin que les ions de ladite matière migrent dans la prothèse.

2. Procédé selon la revendication 1, dans lequel les ions de la seconde espèce ont une énergie supérieure à 10 keV, et le second bombardement est poursuivi jusqu'à ce que la dose d'ions soit au moins égale à celle de la matière qui a été implantée.

3. Procédé selon la revendication 1 ou 2, dans lequel le second bombardement est réalisé à une température à laquelle les trous du réseau cristallin de la matière dont est formée la prothèse sont mobiles.

4. Procédé selon l'une quelconque des revendications 1 et 2, dans lequel les bombardements sont réalisés simultanément.

5. Procédé selon l'une quelconque des revendications précédentes, dans lequel la matière est un métal précieux.

6. Procédé selon l'une quelconque des revendications précédentes, dans lequel la matière est l'argent ou l'or.

7. Procédé selon l'une quelconque des revendications précédentes, dans lequel la seconde espèce ionique est une espèce ionique réactive légère.

8. Procédé selon la revendication 7, dans lequel la seconde espèce ionique est capable de réagir avec la matière de la prothèse.

9. Procédé selon la revendication 8, dans lequel la prothèse est formée de titane.

10. Procédé selon l'une quelconque des revendications précédentes, dans lequel la seconde espèce ionique est N$^+$ ou C$^+$.

**Patentansprüche**

1. Verfahren zur Herstellung einer Prothese mit einem Oberflächenbereich, der bei Gebrauch einer Korrosion oder Erosion unterliegt, das das Unterwerfen dieses Bereiches der Prothese einem Beschuß durch Ionen, die eine vorteilhafte biozide Wirkung aufweisen, wenn sie aus dem Oberflächenbereich der Prothese durch Korrosion oder Erosion bei Gebrauch freigesetzt werden, bis eine Dosis von mindestens 10$^{15}$ Ionen/cm$^2$ des Materials in diesen Bereich der Prothese implantiert worden sind, umfaßt und bei dem man anschließend den implantierten Bereich der Prothese einer Beschießung durch Ionen einer anderen Spezies unterwirft, um zu bewirken, daß die Ionen aus dem Material in die Prothese wandern.

2. Verfahren nach Anspruch 1, bei dem die zweite Ionenspezies eine Energie größer als 10 KeV aufweist und die zweite Beschießung fortgeführt wird, bis eine Ionendosis implantiert wurde, die mindestens der des Materials gleicht.

3. Verfahren nach Anspruch 1 oder 2, bei dem die zweite Beschießung bei einer Temperatur durchgeführt wird, bei der Leerstellen im Kristallgitter des Materials, aus dem die Prothese hergestellt ist, beweglich sind.

4. Verfahren nach einem der Ansprüche 1 bis 2, bei dem die Beschießungen gleichzeitig durchgeführt werden.

5. Verfahren nach einem der vorgehenden Ansprüche, bei dem das Material ein Edelmetall ist.

6. Verfahren nach einem der vorgehenden Ansprüche, bei dem das Material Silber oder Gold ist.

7. Verfahren nach einem der vorhergehenden Ansprüche, bei dem die zweite Ionenspezies eine leicht reaktive Ionenspezies ist.

8. Verfahren nach Anspruch 7, bei dem die zweite Ionenspezies dergestalt ist, daß sie in der Lage ist, mit dem Material der Prothese zu reagieren.

9. Verfahren nach Anspruch 8, bei dem die Prothese aus Titan hergestellt ist.

10. Verfahren nach einem der vorhergehenden Ansprüche, bei dem die zweite Ionenspezies $N^+$ oder $C^+$ ist.

Fig.1(a).

Fig.1(b).